(19) **Europäisches Patentamt / European Patent Office / Office européen des brevets**

(11) **EP 4 260 829 A1**

(12) ## EUROPEAN PATENT APPLICATION
published in accordance with Art. 153(4) EPC

(43) Date of publication:
**18.10.2023 Bulletin 2023/42**

(21) Application number: **21905204.0**

(22) Date of filing: **28.09.2021**

(51) International Patent Classification (IPC):
**A61B 18/14** $^{(2006.01)}$

(86) International application number:
**PCT/CN2021/121398**

(87) International publication number:
**WO 2022/127274 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.12.2020 CN 202011461711**

(71) Applicant: **Shanghai Microport Ep Medtech Co., Ltd.**
**Shanghai 201318 (CN)**

(72) Inventors:
• **MEI, Ying**
**Shanghai 201318 (CN)**
• **LIANG, Bo**
**Shanghai 201318 (CN)**
• **SONG, Yanjun**
**Shanghai 201318 (CN)**
• **SUN, Yiyong**
**Shanghai 201318 (CN)**
• **ZHU, Jing**
**Shanghai 201318 (CN)**

(74) Representative: **Patentship**
**Patentanwaltsgesellschaft mbH**
**Elsenheimerstraße 65**
**80687 München (DE)**

(54) **MEDICAL CATHETER AND MEDICAL DEVICE**

(57) A medical catheter and a medical device, including: a catheter assembly defining a needle channel comprising a distal section with a predefined position; a needle assembly (200) partially disposed in the needle channel and comprising a metal needle (210), the needle assembly (200) movable in an axial direction of the needle channel so that the metal needle (210) passes by or reaches the predefined position; a handle assembly (300) comprising a grip (310) and an advancement member, the advancement member connected to the grip (310) and configured to be moveable relative to the grip (310), the advancement member configured to drive the needle assembly (200) to move along the needle channel; and a first monitoring device configured to sense the arrival of the metal needle (210) at the predefined position and responsively generate a first target signal. During movement of the needle assembly (200) driven by the advancement member away from the grip (310), monitoring on a movement distance of the advancement member relative to the grip (310) is commenced at the time when the metal needle (210) reaches the predefined position as monitored by the first monitoring device. The movement distance can accurately characterize a length of the metal needle (210) that has extended out of the catheter assembly, resulting in improved therapeutic outcomes.

Fig. 12

EP 4 260 829 A1

## Description

## TECHNICAL FIELD

The present invention relates to the field of medical instruments and, in particular, to a medical catheter and a medical device.

## BACKGROUND

Radiofrequency (RF) ablation is a common treatment for arrhythmias. Myocardial scars are the most common substrates that may induce atrial and ventricular arrhythmias. A myocardial scar typically contains viable muscle fiber bundles interspersed in fibrotic tissue, which slow down the conduction of cardiac electrical signals, especially in regions with dense fibrosis where conduction may be even blocked. For myocardial scars with particular fibrotic tissue arrangements, the conduction of cardiac electrical signals through or around these myocardial scars may lead to the generation of reentrant circuits, which may in turn cause arrhythmias. Each of such reentrant circuits includes an isthmus, which location is closely related to a scar boundary zone. A cardiac electrical signal propagates slowly through such an isthmus and then enters the normal myocardium. Ablating the isthmus can improve arrhythmia-related symptoms. For example, it may terminate reentrant tachycardias.

Various techniques such as electroanatomical mapping and activation, entrainment mapping and fifth mapping have been developed in electrophysiological studies so far to identify myocardial scars and potential isthmus locations. Ablation points or lines are then created with radio frequency (RF) energy to break the reentrant circuits. Typically, RF energy is delivered from a tip electrode of a catheter to the endocardial or epicardial surface of the heart, and grounded through electrode pads placed on the patient's skin. In this case, the RF energy is distributed across the tissue between the catheter tip (i.e., the tip electrode) and the electrode pads. At present, standard 7F 4mm ablation catheters have achieved great success in the ablation of reentrant circuits located within a few millimeters of the catheter tip.

Despite the ability to effectively ablate superficial arrhythmia sites, the standard catheters are less effective in ablation at deep intramyocardial or transmural sites. This can be overcome by using charged needle electrodes to deliver RF energy to those sites, and good therapeutic outcomes can be expected. Catheters equipped with an extendible and retractable needle electrode and having the ability of intramyocardial perfusion of a saline solution can also be used to ablate deep intramyocardial circuits. After such a catheter is inserted into a patient's body through a certain path and advanced to a target site in the heart, a handle assembly is manipulated to extend the needle electrode out and a cold saline solution is filled therein at the same time. The needle electrode is inserted into the tissue to access the deep target site and then discharges electrical energy to form a deep ablation lesion there. However, existing such catheters suffer from structural deficiencies and fail to enable an operator to easily determine an extended length of a needle electrode in an accurate manner. Consequently, the needle electrode cannot precisely reach a target site deep in the myocardium, leading to poor therapeutic outcomes or even potential safety hazards.

## SUMMARY OF THE INVENTION

It is an object of the present invention to provide a medical catheter and a medical device. When using the medical catheter for therapeutic treatment, an operator is allowed to accurately determine the time when a metal needle starts being extended out from the medical catheter and a length that it has been extended. As a result, the metal needle can precisely access a target site, resulting in improved therapeutic outcomes.

To this end, the present invention provides a medical catheter, comprising:

a catheter assembly defining a needle channel comprising a distal section with a predefined position;
a needle assembly partially disposed in the needle channel, the needle assembly comprising a metal needle, the needle assembly movable in an axial direction of the needle channel so that the metal needle passes by or reaches the predefined position in the distal section;
a handle assembly comprising a grip and an advancement member, the advancement member connected to the grip and configured to be able to move relative to the grip, the advancement member configured to drive, under the action of an external force, the needle assembly to move in the axial direction of the needle channel; and
a first monitoring device for monitoring whether the metal needle reaches the predefined position, the first monitoring device configured to generate a first target signal upon the metal needle reaching the predefined position.

Optionally, during movement of the needle assembly away from the grip driven by the advancement member, monitoring of a movement distance of the advancement member relative to the grip is started at the time when the metal needle reaches the predefined position.

Optionally, a cross-section of the distal section may have a non-circular shape, wherein a circle inscribed on the cross-section of the distal section matches an outer diameter of the metal needle so that the metal needle is in a coaxial arrangement with the distal section when the metal needle is at least partially located in the distal section.

Optionally, the first monitoring device may comprise a metal contact member, which is disposed at the predefined position of the distal section so as to come into contact with an outer surface of the metal needle and be electrically connected to the metal needle, thereby enabling the generation of the first target signal.

Optionally, a ring-shaped first accommodating groove may be defined in a wall of the distal section at the predefined position, wherein the metal contact member is a ring-shaped structure defining a first inner bore, wherein the metal contact member is fitted in the first accommodating groove, and wherein the first inner bore has a cross-section matching the cross-section of the distal section in terms of both shape and size.

Optionally, the catheter assembly may comprise a catheter body, a tip electrode and a limiting member, the tip electrode disposed at a distal end of the catheter body, the tip electrode defining a second inner bore extending axially therethrough, the limiting member configured as an insulator disposed in the second inner bore, the limiting member defining a third inner bore extending axially therethrough, the third inner bore making up the distal section of the needle channel.

Optionally, a plurality of ridges extending in an axial direction of the limiting member may be provided on a wall of the third inner bore and spaced apart across a circumference of the limiting member, wherein in a cross-section of the third inner bore, each of the ridges has a vertex located on a circle inscribed on the third inner bore.

Optionally, the needle assembly may further comprise a first fluid tube connected at a proximal end thereof to the advancement member and at a distal end thereof to the metal needle, wherein

the metal needle, the first monitoring device and the tip electrode are electrically connected to an external device by separate wires, in which the wire connecting the first monitoring device and the tip electrode are provided in the catheter assembly.

Optionally, the first fluid tube may define a first lumen, wherein

the catheter body comprises an outer sleeve, a second fluid tube and an external adapter tube, the second fluid tube partially disposed in the outer sleeve, the second fluid tube extending in an axial direction of the outer sleeve, the second fluid tube connected at a proximal end thereof to the handle assembly, the second fluid tube configured to be stationary relative to the grip, the second fluid tube defining a second lumen for insertion of the first fluid tube therein so that a fluid channel is defined between the first fluid tube and the second fluid tube, the fluid channel separated from the first lumen, the second fluid tube provided in its wall around a proximal end thereof with a perfusion aperture in communication with the fluid channel, the external adapter tube in communication with the perfusion aperture, a distal end of the second fluid tube connected to a proximal end of the tip electrode, the second fluid tube in communication with the third inner bore.

Optionally, the first monitoring device may comprise an infrared sensor disposed at the predefined position of the distal section.

Optionally, the handle assembly may further comprise a manipulation member rotatably disposed at a proximal end of the grip, wherein the advancement member comprises a threaded rod having a distal end portion disposed inside the grip and connected to a proximal end of the needle assembly, the threaded rod configured to be circumferentially stationary relative to the grip and axially movable relative to the grip, a proximal end of the threaded rod spirally fitted to the manipulation member so as to be spirally rotatable.

Optionally, the threaded rod or the grip may be provided thereon with graduation lines, at least some of which are visible from the outside, thereby enabling monitoring a movement distance of the threaded rod relative to the grip. Alternatively, the manipulation member may be provided thereon with graduation lines, wherein an angle of rotation of the manipulation member relative to the grip is monitored, and a movement distance of the threaded rod relative to the grip is monitored based on a fixed ratio of the angle of rotation to a displacement distance of the threaded rod.

Optionally, the medical catheter may further comprise a second monitoring device for producing a second target signal when the threaded rod moves relative to the grip, based on which the movement distance of the threaded rod relative to the grip is calculated.

Optionally, the second monitoring device may comprise a first portion and a second portion, the first portion connected to the manipulation member and maintained axially stationary relative to the grip, the second portion connected to the threaded rod so as to be axially movable relative to the grip, the first and second portions cooperating with each other to together make up a sliding rheostat, which is connected in a circuit and configured to produce an electrical signal serving as the second target signal.

Optionally, the first portion may comprise a metal base and a resistance wire, the metal base implemented as an elongate structure extending in an axial direction of the threaded rod, the metal base connected at a proximal end thereof to the manipulation member so that the first portion is axially stationary relative to the grip, the resistance wire wound on an outer surface of the metal base, the second portion comprising a sliding tab in contact with the resistance wire, the sliding tab configured to slide on the resistance wire in response to movement of the threaded rod relative to the grip, causing changes in a resistance of the sliding rheostat and hence in the electrical signal.

Optionally, a second accommodating groove may be formed in a proximal end face of the threaded rod to receive the second portion, wherein the threaded rod further defines a channel, which extends axially and communicates with the second accommodating groove, and in which the first portion is inserted.

The present invention also provides a medical device comprising a control unit and the medical catheter as defined above. The control unit comprises a first signal acquisition module and an indication module. The first signal acquisition module is communicatively connected to the first monitoring device and the metal needle and configured to collect the first target signal. The indication module is configured to indicate, based on the first target signal, that the metal needle reaches the predefined position.

The present invention also provides another medical device comprising a control unit and the medical catheter as defined above. The medical device is characterized in that the control unit comprises a first signal acquisition module, an indication module, an electrical signal generation module, a second signal acquisition module and a calculation module.

The first signal acquisition module is communicatively connected to the first monitoring device and the metal needle and configured to collect the first target signal. The indication module configured to indicate, based on the first target signal, that the metal needle reaches the predefined position.

The electrical signal generation module is configured to power the second monitoring device, and the second signal acquisition module is communicatively connected to the second monitoring device and configured to collect the second target signal. The calculation module is configured to calculate, based on the second target signal, a movement distance of the advancement member relative to the grip.

Optionally, the control unit may be configured so that the first signal acquisition module collects the first target signal and sends it to the indication module and the electrical signal generation module and the electrical signal generation module responsively powers the second monitoring device and that the second signal acquisition module collects the second target signal and sends it to the calculation module and the calculation module responsively calculates the movement distance of the advancement member relative to the grip based on the second target signal.

Optionally, the control unit may further comprise a storage unit in which a fixed distance from a proximal end of the first monitoring device to a distal end of the needle channel is pre-stored, wherein the calculation module is configured to calculates a difference between the movement distance and the fixed distance, which is used to characterize an extended length of the needle coming out from the medical catheter.

Compared with the prior art, the medical catheter and medical device of the present invention have the following advantages: the medical catheter includes a catheter assembly, a needle assembly, a handle assembly and a first monitoring device, the catheter assembly defining a needle channel comprising a distal section with a predefined position, the needle assembly partially disposed in the needle channel, the needle assembly comprising a metal needle, the needle assembly movable in an axial direction of the needle channel so that the metal needle passes by or reaches the predefined position of the distal section, the handle assembly comprising a grip and an advancement member, the advancement member connected to the grip and configured to be moveable relative to the grip, the advancement member configured to drive, under the action of an external force, the needle assembly to move in the axial direction of the needle channel, the first monitoring device configured to monitor whether the metal needle reaches the predefined position and generate a first target signal upon the metal needle reaching the predefined position. During movement of the needle assembly driven by the advancement member away from the grip, monitoring of a movement distance of the advancement member relative to the grip is started at the time when the metal needle reaches the predefined position, and the difference between the movement distance and a fixed distance from a proximal end of the first monitoring device to a distal end of the needle channel is calculated and used to characterize an extended length of the metal needle. When the medical catheter bends, it is difficult to ensure coaxiality of the needle assembly with the needle channel across the full extent of the needle channel. Consequently, when moving the advancement member distally to advance the metal needle out of the needle channel, the metal needle may not move in synchronization with the advancement member. When this happens, if a movement distance of the advancement member relative to the grip is directly taken as a basis for determining a length of the metal needle that has extended out of the catheter assembly (i.e., an extended length), the determined extended length will be surely inaccurate and metal needle cannot be precisely inserted into tissue to access a predetermined position therein. In contrast, according to the present invention, the time when the metal needle reaches the predefined position in the distal section of the needle channel as monitored by the first monitoring device is taken as a time when the metal needle starts being extended, and just at this time, monitoring of a movement distance of the advancement member relative to the grip is commenced. The difference between the monitored movement distance and the fixed distance from the proximal end of the first monitoring device to the distal end of the needle channel is calculated and used to characterize an extended length of the metal needle. In other words, according to the present invention, monitoring of a movement distance of the advancement member when the metal needle does not move in synchronization with it is given up, and only its movement distance during movement of the metal needle in synchronization with it is monitored. This enables accurate extended length determination, which can result in improved therapeutic outcomes.

## BRIEF DESCRIPTION OF THE DRAWINGS

The accompanying drawings are provided to facilitate a better understanding of the present invention and do not unduly limit the scope thereof in any sense.

Fig. 1 is a schematic diagram showing the structure of a medical catheter according to an embodiment of the present invention.

Fig. 2 is a schematic diagram showing the structure of a needle assembly in a medical catheter according to an embodiment of the present invention.

Fig. 3 is a cross-sectional view of the medical catheter of Fig. 1 taken along N-N.

Fig. 4 is a cross-sectional view of the medical catheter of Fig. 1 taken along F-F.

Fig. 5 is a cross-sectional view of the medical catheter of Fig. 1 taken along S-S.

Fig. 6 is a schematic diagram showing the structure of a limiting member in a medical catheter according to an embodiment of the present invention.

Fig. 7 is a schematic view of the limiting member in the medical catheter of Fig. 6, viewed in the direction from its distal to proximal end.

Fig. 8 is a cross-sectional view of the limiting member in the medical catheter of Fig. 6 taken along A-A.

Fig. 9 schematically illustrates how first and second fluid tubes in a medical catheter cooperate with each other according to an embodiment of the present invention.

Fig. 10 is a schematic cross-sectional view of a metal contact member in a medical catheter according to an embodiment of the present invention.

Fig. 11 is a front view of a metal contact member in a medical catheter according to an embodiment of the present invention.

Fig. 12 schematically illustrates how a limiting member and a metal contact member in a medical catheter are assembled together according to an embodiment of the present invention.

Fig. 13 is a schematic diagram showing the structure of a portion of a medical catheter according to an embodiment of the present invention, in which the structure of a second monitoring device is also shown.

Fig. 14 is a cross-sectional view of the medical catheter of Fig. 1 taken along V-V.

Fig. 15 schematically illustrates how a movement distance of an advancement member in a medical catheter is calculated according to an embodiment of the present invention.

In these figures, 110 denotes a tip electrode; 111a, a second inner bore; 111b, a first blind hole; 111c, a second blind hole; 120, a limiting member; 121, a third inner bore; 122, ridges; 123, a first accommodating groove; 130, an outer sleeve; 131, a main section; 132, a controlled bendable section; 132a, a fourth lumen; 132b, a fifth lumen; 132c, a sixth lumen; 132d, a seventh lumen; 140, a second fluid tube; 141, a perfusion aperture; 200, a needle assembly; 210, a metal needle; 220, a first fluid tube; 300, a handle assembly; 310, a grip; 311, limiting grooves; 320, a threaded rod; 321, a threaded rod body; 322, a locating block; 323, limiting sliders; 330, a manipulation member; 410, a metal contact member; 411, a first inner bore; 412, a wire connecting the metal contact member; 500, a second monitoring device; 511, a metal base; 512, a resistance wire; and 520, a sliding tab.

## DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or" unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

Objects, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In the drawings, like reference numbers indicate identical or similar elements.

As used herein, the terms "proximal" and "distal" are intended to refer to relative orientations, relative positions and directions between components of a medical device or actions thereof, as viewed by a physician operating the device. Without wishing to be limiting, a "proximal end" usually refers to an end closer to the operator, and a "distal end" to an end first enters the body of a patient, during normal operation of the medical device.

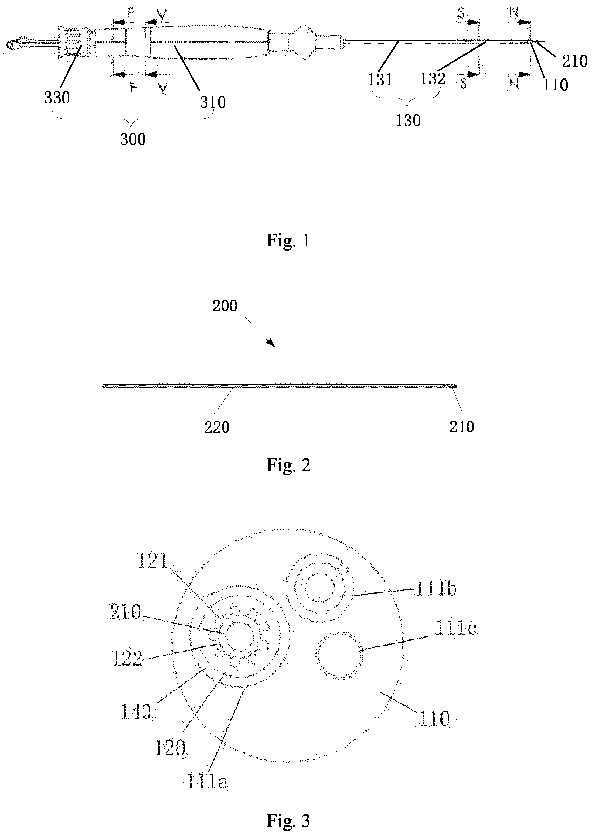

As shown in Figs. 1 to 4, in embodiments of the present invention, there is provided a medical catheter including a catheter assembly, a needle assembly 200, a handle assembly 300 and a first monitoring device. The catheter assembly defines a needle channel including a distal section having a predefined position. The needle assembly 200 is partially disposed in the needle channel and includes a metal needle 210. The needle assembly 200 is movable in an axial direction of the needle channel so that the metal needle 210 can pass by or rest at the predefined position of the distal section. The handle assembly 300 includes a grip 310 and an advancement member. The advancement member is connected to the grip 310 and configured to be able to move relative to the grip 310. The advancement member is used to drive, under the action of an external force, the needle assembly 200 to move in the axial direction of the needle channel. The first monitoring device is used to monitor whether the metal needle 210 arrives at the predefined position and is configured to generate a first target signal upon the metal needle reaching the predefined position.

The catheter assembly may bend when a distal end of the medical catheter reaches a target site in a patient's body during its advancement in a blood vessel. When this happens, the needle assembly 200 may lose its coaxiality with the needle channel across the full extent of the needle channel, and friction may occur between the needle assembly 200 and a wall of the needle channel. Moreover, depending on the bending condition of the catheter assembly, the friction may occur at different magnitudes in various directions, possibly leading to asynchronous movement of the metal needle 210 and the advancement member. For example, in order to drive movement of the needle assembly 200 away from the grip 310, the advancement member first moves a certain distance before the metal needle 210 can move in synchronization therewith. That is, the movement of the metal needle 210 is delayed relative to that of the advancement member. Therefore, if the advancement member is monitored for its movement distance as soon as it starts to move and a movement distance of the metal needle 210 is considered to be equal to the monitored movement distance of the advancement member, an extended length of the metal needle 210 derived from the movement distance thereof will be surely inaccurate. Here, the "movement of the needle assembly 200 away from the grip 310" refers to movement of the needle assembly 200 from its proximal to distal end. On the contrary, movement of the needle assembly 200 toward the grip 310 refers to its movement from the distal to proximal end. The "extended length" refers to a length of a portion of the metal needle 210 that has been extended out of the needle channel.

In view of this, in this embodiment, the extended length is determined as follows: as monitored by the first monitoring device, upon the metal needle 210 reaching the predefined position during movement of the needle assembly 200 away from the grip 310 under the drive of the advancement member, monitoring of a movement distance of the advancement member relative to the grip 310 is started, and the extended length of the metal needle 210 is characterized by the distance between the monitored movement distance and a fixed distance from a proximal end of the first monitoring device to a distal end of the needle channel. In other words, according to this embodiment, the first monitoring device is used to monitor the arrival of the metal needle 210 at the predefined position, and the time when the metal needle 210 reaches the predefined position is taken as a start time for monitoring the movement distance of the advancement member relative to the grip 310. In this way, inaccurate extended length determination attributable to the delayed movement of the metal needle 210 can be avoided. According to this embodiment, the medical catheter may be a myocardial injection catheter, an ablation catheter or another type of catheter, without departing from the scope of the present invention. The following description of structural details of the medical catheter is set forth in the exemplary context of an ablation catheter. It would be appreciated that, in this embodiment, the metal needle 210 is a hollow structure, and the needle assembly 200 may further include a first fluid tube 220, which is connected at a distal end thereof to the metal needle 210 and defines a first lumen extending axially therethrough and communicating with the metal needle 210. Additionally, the first fluid tube 220 is connected at a proximal end thereof to the advancement member.

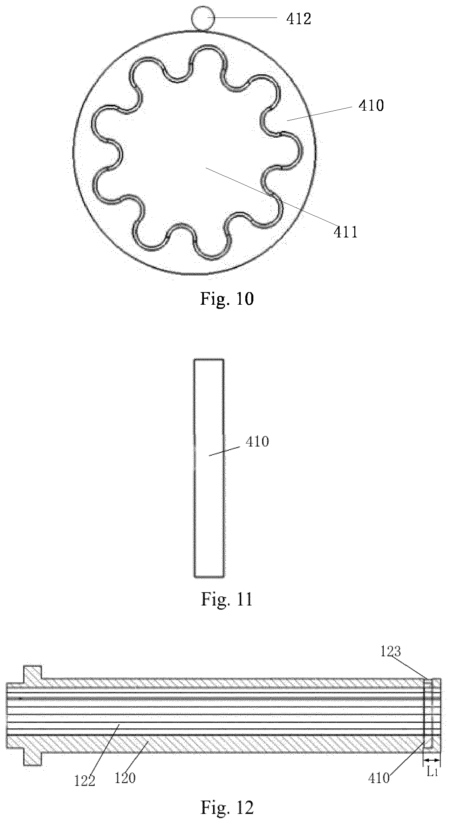

In this embodiment, the first monitoring device includes a metal contact member 410 (see Figs. 10 to 12). The metal contact member 410 is disposed at the predefined position of the distal section and is configured to come into contact with an outer surface of the metal needle 210 and be thereby electrically connected to the metal needle 210, thus enabling the generation of the first target signal.

A proximal end of the metal needle 210 may be electrically connected by wires to an external first signal acquisition module and other external device(s), and the metal contact member 410 is also wired to an external device. When the metal needle 210 reaches the predefined position and comes into contact with the metal contact member 410, it is electrically connected to the metal contact member 410, leading to an instantaneous resistance increase in a first circuit formed by the two and wires. As a result, an electrical signal (i.e., the first target signal, which is, for example, a current signal) is produced and collected by the first signal acquisition module. The first signal acquisition module may be communicatively connected to an indication module. In response to the collection of the first target signal at the first signal acquisition module, the indication module may provide an indication to an operator by means of sound, light, vibration, etc. and may record the time as a start time for monitoring movement of the advancement member relative to the grip 310.

Below, structural details of the various components of the medical catheter and how they operate with one another will be described in detail with reference to the accompanying drawings.

With continued reference to Figs. 1 and 3, the catheter assembly includes a catheter body, a tip electrode 110 and a limiting member 120. The tip electrode 110 is disposed at a distal end of the catheter body, and the limiting member 120 is disposed within the tip electrode 110.

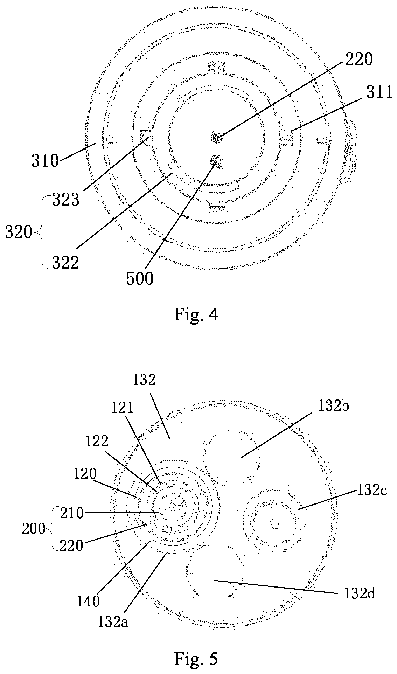

With particular reference to Fig. 3, in conjunction with Figs. 1 and 5, the catheter body includes an outer sleeve 130 and a second fluid tube 140. The second fluid tube 140 is disposed within the outer sleeve 130 and defines a second lumen extending axially therethrough. The second lumen is configured for insertion of the first fluid tube 220 therein so that a fluid channel is defined between an inner wall surface of the second fluid tube 140 and an outer wall surface of the first fluid tube 220 and is separated from the first lumen of the first fluid tube 220. Proximal ends of the outer sleeve 130 and the second fluid tube 140 are both connected to the handle assembly 300 and configured to be maintained stationary relative to the grip 310. The tip electrode 110 is connected to both a distal end of the outer sleeve 130 and a distal end of the second fluid tube 140. The tip electrode 110 defines a second inner bore 111a extending axially therethrough. The distal end of the second fluid tube 140 and the limiting member 120 are disposed within the second inner bore 111a, and the limiting member 120 defines a third inner bore 121 extending axially therethrough. The third inner bore 121 communicates with the second lumen of the second fluid tube 140, and they together make up the needle channel. The third inner bore 121 provides the aforementioned distal section of the needle channel. In other words, the predefined position is defined in the third inner bore 121.

In operation, the advancement member drives, under the action of an external force, the metal needle 210 to advance or retreat within the third inner bore 121 of the limiting member 120. Here, by "advance or retreat", it is intended to mean that the metal needle 210 moves in a direction from its proximal to distal end (i.e., away from the grip 310) to extend out of the needle channel, or in a direction from its distal to proximal end (i.e., toward the grip 310) to withdraw into the needle channel. It would be appreciated by those skilled in the art that, in order to avoid the occurrence of a discharge, or the establishment of an electrical connection, between the metal needle 210 and the tip electrode 110, the limiting member 120 is configured as an insulator and has the same length as the tip electrode 110 in the axial direction. In this way, the metal needle 210 is insulated from the tip electrode 110.

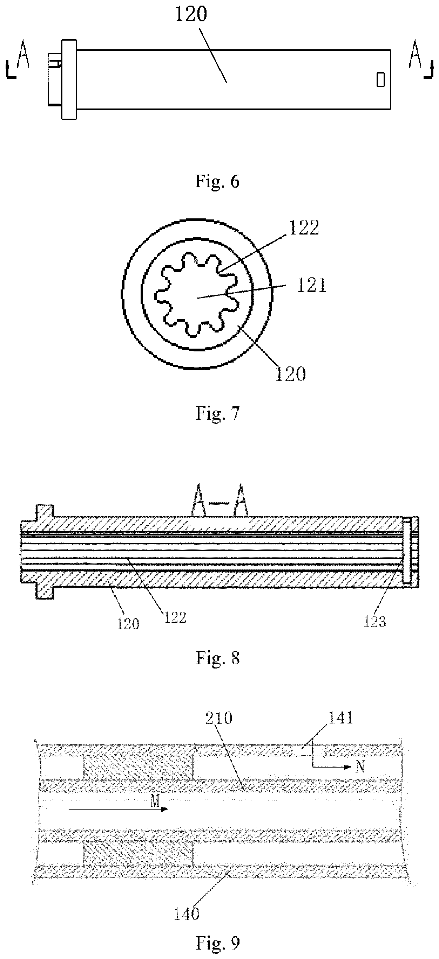

In an example where the medical catheter is implemented as an ablation catheter, after the metal needle 210 extends out of the needle channel and pierces to a target site, RF energy may be applied to tissue at the target site to form an ablation lesion. At this time, the operator may fill a fluid medium such as a physiological saline solution in the first lumen of the first fluid tube 220 to cool the metal needle 210. In the first lumen of the first fluid tube 220, the fluid medium may flow in a path as shown by the arrow M in Fig. 9. In addition, the operator may perfuse a fluid medium such as a physiological saline solution through the fluid channel of the second fluid tube 140 to wash a target region with the fluid medium to avoid thrombosis. In the fluid channel of the second fluid tube 140, the fluid medium may flow in a path as shown by the arrow N in Fig. 9. Therefore, preferably, the limiting member 120 is kept concentric with the metal needle 210, with a gap being left between its inner wall surface and an outer wall surface of the metal needle 210 for passage therethrough of the physiological saline solution exiting the second fluid tube 140. To this end, the third inner bore 121 of the limiting member 120 is configured to have a non-circular cross-sectional shape (i.e., the distal section has a non-circular cross-sectional shape), and a circle inscribed thereon matches an outer diameter of the metal needle 210. In this way, when at least partially positioned in the distal section of the needle channel, the metal needle 210 is in a coaxial arrangement with the distal section. In this arrangement, an axis of the limiting member 120 coincides with an axis of the circle inscribed on the cross-section. Accordingly, in this embodiment, the description that "the metal needle 210 is in a coaxial arrangement with the distal section" intends to mean that the metal needle 210 is coaxial with the circle inscribed on the cross-section of the limiting member 120.

In an illustrative embodiment, referring to Figs. 6 to 8, a plurality of ridges 122 may be raised from a wall of the third inner bore 121. These ridges may extend in an axial direction of the limiting member 120 and spaced apart across a circumference of the limiting member 120. With particular reference to Fig. 7, in the cross-section of the limiting member 120, each ridge 122 defines a peak, and each two adjacent ridges 122 define a valley therebetween. Each peak provides radial support to the metal needle 210. When vertices of all the peaks are situated on the circle inscribed on the cross-section of the third inner bore 121, each peak will provide equal support to the metal needle 210, which can maintain the metal needle 210 in coaxiality with the inscribed circle. Moreover, each valley provides an outflow channel for the physiological saline solution. In an alternative embodiment, the cross-sectional shape of the third inner bore 121 may be rectangular, elliptic or otherwise shaped, without departing from the scope of the present invention.

It is to be noted that in order to enable the perfusion with the fluid medium through the second fluid tube 140, the catheter assembly may further include an external adapter tube (not shown). As shown in Fig. 9, a perfusion aperture 141 in communication with the fluid channel is formed in a wall of the second fluid tube 140 around the proximal end thereof. The external adapter tube may communicate with the perfusion aperture 141 and proximally extend out of the handle assembly 300. Further, on a proximal side of the perfusion aperture 141, the outer wall surface of the first fluid tube 220 may be sealingly connected to the inner wall surface of the second fluid tube 140.

Referring back to Fig. 8, in conjunction with Figs. 10 to 12, a first accommodating groove 123 may be formed in a wall of the third inner bore 121 in the limiting member 120 at the predefined position. The metal contact member 410 may be designed as a ring fitted in the first accommodating groove 123. The metal contact member 410 may define a first inner bore 411, which extends axially therethrough and has a cross-section matching the cross-section of the third inner bore 121 in terms of both shape and size. As the metal needle 210 has a distal beveled edge that is angled with respect to an axis thereof (see Fig. 2) in order to facilitate its piercing of tissue and access to the target site, this configuration of the metal contact member 410 enables the first monitoring device to immediately identify the distal end of the metal needle 210 as soon as it reaches the predefined position. In addition, this configuration enables a larger contact area between the metal contact member 410 and the metal needle 210, which makes the generated first target signal more stable and improves the accuracy of extended length monitoring. The present application is not limited to any particular shape of the metal contact member 410, as long as it enables at least partial contact of the metal contact member 410 with the metal needle 210 and hence the establishment of a current connection. For example, in alternative implementations, the metal contact member 410 may be a block disposed on the wall of the third inner bore so as to be inwardly raised therefrom, or the metal contact member 410 may include several contact blocks spaced apart across a circumference of the third inner bore.

Optionally, as shown in Fig. 12, the metal contact member 410 may be provided at a distal end of the limiting member 120. The metal contact member 410 may have a predetermined axial length, and the first target signal may be generated as soon as the distal end of the metal needle 210 comes into contact with the metal contact member. Accordingly, the predefined position may be provided at a proximal end of the metal contact member 410.

Assuming that the fixed distance from the proximal end of the metal contact member 410 to the distal end of the needle channel is $L_1$, if the metal needle 210 extends out of the needle channel from the distal end thereof as a result of the needle assembly 200 being advanced by the advancement member and the advancement member further moves a distance $L_2$ relative to the grip 310 after the first target signal is generated, then an extended length of the metal needle 210 will be equal to the difference between L2 and L1.

The metal needle 210 and the metal contact member 410 are connected to separate wires (the wire connected to the metal needle 210 is not shown, and that connected to the metal contact member is indicated as 412 in Fig. 10). Likewise, the tip electrode 110 is connected to an external device by a wire. Additionally, the catheter assembly may be distally bendable under the control of a traction thread. In this embodiment, the wire connected to the metal needle 210 may be received in the first lumen of the first fluid tube 220 (the wire may be provided on an outer surface thereof with a protective sleeve), and the wires connected to the metal contact member 410 and the tip electrode 110 and a control wire may be all disposed within the catheter assembly. In this way, the wires will not occupy internal spaces of the needle assembly 200 and be prevented from breakage or disconnection during filling of fluid into the needle assembly 200. In other embodiments, in order to prevent breakage or disconnection of the wire connected to the metal needle 210 in the first lumen of the first fluid tube 220, it may be alternatively disposed outside the first lumen. Of course, the present application is not limited to any particular locations or arrangements of the wires connected to the metal needle 210, the metal contact member 410 and the tip electrode 110.

For example, referring back to Figs. 1 and 5, the outer sleeve 130 may be a tube consisting of multiple sections including a main section 131 and a controlled bendable section 132, which are joined together axially. The main section 131 may be proximally connected to the grip 310 in the handle assembly 300 and define a third lumen extending axially therethrough. The controlled bendable section 132 may be arranged distally to the main section 131 and bendable under the control of the traction thread. The controlled bendable section 132 may define multiple lumens therein, preferably a fourth lumen 132a, a fifth lumen 132b, a sixth lumen 132c and a seventh lumen 132d, which all extend axially through the controlled bendable section 132 and are separated from one another. In other words, the fourth lumen 132a, the fifth lumen 132b, the sixth lumen 132c and the seventh lumen 132d may separately communicate with the third lumen. Referring again to Fig. 3, the tip electrode 110 may further define a first blind hole 111b and a second blind hole 111c, both separated from the second inner bore 111a.

During assembly of the catheter, as the fourth lumen 132a of the controlled bendable section 132 may be brought into communication with the third inner bore 121 of the limiting member 120, allowing the second fluid tube 140 to be distally passed through the second lumen of the main section 131 into the fourth lumen 132a of the controlled bendable section 132 and further to a proximal end of the tip electrode 110 and connected thereto. As a result, the second lumen is brought into communication with the third inner bore 121 of the limiting member 120, and the two together make up the needle channel. The fifth lumen 132b of the controlled bendable section 132 may be used to accommodate the wire connected to the tip electrode 110, and the sixth lumen 132c may be used to accommodate the traction thread. The seventh lumen 132d may be used to accommodate a wire connected to a sensor, such as a magnetic sensor for identifying the position of the catheter body in operation. The first blind hole 111b may be used to receive and anchor a distal end of the traction thread and a distal end of the wire connected to the tip electrode 110. The second blind hole 111c may be used to receive the wire connected to the sensor. It would be appreciated that the catheter assembly may further include a ring electrode disposed over the outer sleeve. In this case, the fourth lumen 132a may be further used to accommodate a wire connected to the ring electrode, and the first blind hole 111b may be further used to receive and anchor a distal of the wire connected to the ring electrode.

In an alternative embodiment, the first monitoring device may further include an infrared sensor, which may include a transmitter and a receiver, disposed respectively on opposite sides of the predefined position in the limiting member 120. When the metal needle 210 reaches the predefined position, it may be located between the transmitter and the receiver, making the receiver unable to receive an infrared signal, i.e., leading to an interruption in the reception of the infrared signal. This could be taken as the first target signal.

As would be understood from the above description, in response to the first target signal being generated by the first monitoring device, it can be known that the metal needle 210 has reached the predefined position, and the time when this happens can be used to characterize a time when the metal needle 210 starts being extended. Just at this time, monitoring of a movement distance of the advancement member relative to the grip 310 can be started, and the difference between the monitored movement distance and the aforementioned fixed distance can be used to characterize an extended length of the metal needle 210.

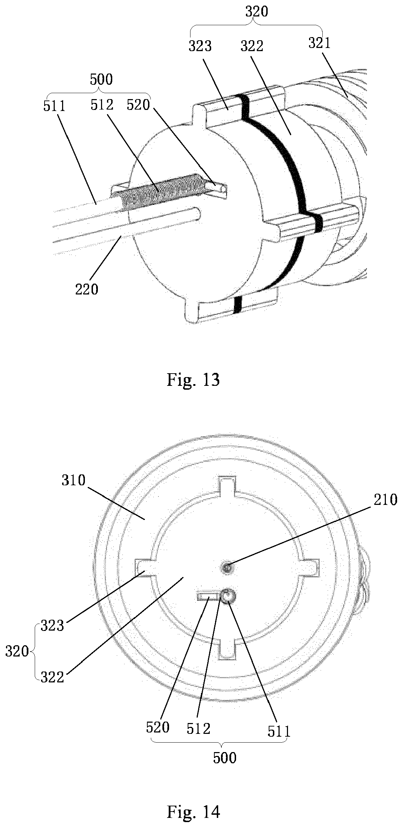

Referring back to Fig. 4, in conjunction with Figs. 1 and 14, the advancement member may include a threaded rod 320, which is distally disposed within the grip 310 and configured to be able to move axially relative to the grip 310. For example, the grip 310 may define an eighth lumen extending axially therethrough, and a plurality of limiting grooves 311 spaced apart from one another may be formed in a wall of the eighth lumen. The threaded rod 320 may include a threaded rod body 321 and a locating block 322 disposed at a distal end of the threaded rod body 321. The locating block 322 may be provided on an outer wall surface thereof with limiting sliders 323 received in the limiting groove 311 so as to be axially movable therein. The threaded rod 320 may define a ninth lumen extending axially therethrough, and the first fluid tube 220 may proximally pass through the ninth lumen so as to proximally extend outside of the ninth lumen from a proximal end thereof. Moreover, first fluid tube 220 may axially move in synchronization with the threaded rod 320. The handle assembly 300 may further include a manipulation member 330, which may be rotatably disposed at a proximal end of the grip 310. In particularly, it may be spirally fitted with the proximal end of the grip 310 so as to be spirally rotatable. As a result of the manipulation member 330 being rotated in a first direction, the threaded rod may drive the needle assembly 200 to move away from the grip 310. Rotation of the manipulation member 330 in a second direction may cause the threaded rod to drive the needle assembly 200 to move toward the grip 310. The second direction is opposite to the first direction. That is, when the first direction is clockwise, the second direction is counterclockwise; and vice versa.

In some embodiments, the threaded rod 320 may be graduated with lines, at least some of which may be visible from the outside. For example, a proximal end of the threaded rod may pass through the manipulation member and extend out of it from a proximal end thereof so that those of the graduation lines on this extension of the threaded rod that is exposed outside of the manipulation member are visible from the outside (not shown). Alternatively, the grip may be provided with a window, through which at least some of the graduation lines are visible from the outside. A transparent cover (not shown) may be provided within the window. As such, the operator may monitor a movement distance of the threaded rod relative to the grip by observing the graduation lines on the threaded rod. In still alternative embodiments, the grip may have a visual portion (e.g., made of a transparent material or the like) encompassing at least the whole axial movement range of the threaded rod, and the graduation lines may be provided in the visual area. The operator may monitor a movement distance of the threaded rod relative to the grip by observing graduation lines that the threaded rod has passed by. However, the present application is not limited to these arrangements of the graduation lines. In some further embodiments, the graduation lines may be provided on the manipulation member (not shown), and a movement distance of the threaded rod relative to the grip may be monitored by observing an angle that the manipulation member has been rotated. This is because there is a fixed conversion relationship between the angle of rotation of the manipulation member and an actual displacement distance of the threaded rod. That is, the actual displacement distance of the threaded rod can be derived from the angle of rotation and the fixed conversion relationship, and the movement distance of the threaded rod relative to the grip can be in turn calculated from the actual movement distance.

Alternatively, as shown in Fig. 4, the medical catheter may further include a second monitoring device 500, which may monitor movement of the threaded rod 320 relative to the grip 310 and produce a second target signal. A movement distance of the threaded rod 320 relative to the grip 310 may be calculated from the second target signal. As shown in Figs. 13 and 14, the second monitoring device 500 may include a first portion and a second portion. The first portion may be connected to the manipulation member (not shown in Figs. 13 and 14), and the second portion may be connected to the threaded rod 320 so as to be movable in synchronization with the threaded rod 320. The first and second portions may cooperate with each other to together function like a sliding rheostat. The sliding rheostat may be connected to a power supply so that the two form a second circuit capable of producing an electrical signal serving as the second target signal. When the threaded rod 320 is moving, the resistance of a portion of the sliding rheostat connected in the second circuit may vary, resulting in variation of the electrical signal. A movement distance of the threaded rod 320 relative to the grip 310 may be calculated from the variation of the electrical signal.

In particular, the first portion may include a metal base 511 and a resistance wire 512. The metal base 511 may be an elongate structure extending in an axial direction of the threaded rod 320. Moreover, a proximal end of the metal base 511 may be connected to the manipulation member 330. The resistance wire 512 may be wound on an outer surface of the metal base 511. The second portion may include a sliding tab 520, which is connected to the threaded rod 320 and movable in synchronization with the threaded rod 320. The sliding tab 520 may be always kept in contact with the resistance wire 512.

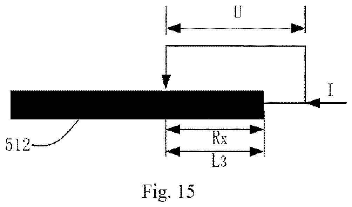

In this way, the power supply, the resistance wire 512 and the sliding tab 520 may form the second circuit. Given a constant voltage U being provided by the power supply, a current I flowing in the second circuit will

be: $= \dfrac{U}{R_x}$ , where $R_x$ represents the resistance in the second circuit, which is determined by a length $L_3$ of the resistance wire 512 connected in the second circuit (see Fig. 15).

When the manipulation member 330 is rotated to drive the threaded rod 320 to move relative to the grip 310 and hence to drive the needle assembly 200 to move away from the grip 310 so that the metal needle 210 is at least partially extended from the needle channel, the first portion remains stationary relative to the grip 310 in the axial direction, while the sliding tab 520 moves axially along with the threaded rod 320. As a result, an increased length of the resistance wire 512 is connected in the second circuit, increasing the resistance in the second circuit and reducing the current flowing through the sliding rheostat. In case of the electrical signal being implemented as the current, at any time, the length of the resistance wire 512 connected in the second circuit can be calculated from the current in the second circuit and the resistivity of the resistance wire 512, and a movement distance of the threaded rod 320 relative to the grip 310 can be derived from the length.

Preferably, the sliding rheostat is arranged within the handle assembly 300. With particular reference to Fig. 14, a second accommodating groove may be formed at the proximal end of the threaded rod 320 to receive the second portion, and the first portion may be inserted in a channel in the threaded rod, which communicates with the second accommodating groove.

Alternatively, the resistance wire 512 may be embedded in the threaded rod 320 so as to be axially movable in synchronization with the threaded rod 320. The sliding tab 520 may be fixed to the metal base 511 so as to be always kept in contact with the resistance wire 512. Both the sliding tab 520 and the metal base 511 may be maintained stationary relative to the grip 310. In this way, the power supply, the resistance wire 512 and the sliding tab 520 can also form the second circuit, and a movement distance of the threaded rod 320 relative to the grip 310 can be derived in the same manner as described above.

The present invention embodiment further includes a medical device including the medical catheter as defined above and a control unit. The control unit includes a first signal acquisition module and an indication module. The first signal acquisition module is communicatively connected to the first monitoring device and the metal needle and configured to collect the first target signal, and the indication module is used to indicate, based on the first target signal, that the metal needle reaches the predefined position.

When the medical catheter further includes the second monitoring device, the control unit may further include an electrical signal generation module, a second signal acquisition module and a calculation module. The electrical signal generation module may be used to power the second monitoring device, and the second signal acquisition module may be used to collect the second target signal. The calculation module may be used to calculate a movement distance of the advancement member relative to the grip from the second target signal.

Accordingly, during use of the medical device, an extended length of the metal needle may be monitored in the following manner. An external force is applied to the advancement member to drive the advancement member to move relative to the grip and to cause the needle assembly move away from the grip. As monitored by the first monitoring device, upon the metal needle being detected as reaching the predefined position, the first target signal is produced. The first signal acquisition module collects the first target signal and sends it to the indication module and the electrical signal generation module, and the electrical signal generation module responsively powers the second monitoring device and produces the second target signal. The second signal acquisition module collects the second target signal and passes it on to the calculation module, which then calculates, based on the second target signal, a movement distance of the threaded rod relative to the grip.

It would be appreciated that the control unit may further include a storage unit in which the fixed distance from the proximal end of the first monitoring device to the distal end of the needle channel may be stored in advance. Accordingly, the calculation module may be used to calculate the difference between the movement distance of the threaded rod relative to the grip and the fixed distance, which characterizes an extended length of the metal needle.

In embodiments of the present invention, the first signal monitoring device is used to sense the arrival of the metal needle at the predefined position, and the time of arrival is taken as a time when the metal needle starts being extended. Moreover, just at this time, monitoring of a movement distance of the advancement member relative to the grip is commenced, and the difference between the monitored movement distance and the fixed distance is utilized to characterize an extended length of the metal needle. In this way, interference of a movement distance of the advancement member resulting from movement of the advancement member before synchronization of movement is established between the metal needle and the advancement member with the calculation of the extended length can be eliminated, thus enable accurate calculation of the extended length. As a result, the metal needle can precisely access a target site, and a penetration depth of the metal needle before it reaches the target site can be accurately determined. These can result in improved therapeutic outcomes.

Although the innovation of the present invention comes out from the field of electrophysiological RF ablation technology, those skilled in the art will understand that the present invention can be applied to ablation needles in other fields and injection needles not used for ablation, such as myocardial injection needles. Indeed, the concept of the present invention can be used in any application requiring accurate extended length determination, but some structural adaptations may be necessary.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical catheter, comprising:

   a catheter assembly defining a needle channel, the needle channel comprising a distal section with a predefined position;
   a needle assembly partially disposed in the needle channel, the needle assembly comprising a metal needle, the needle assembly movable in an axial direction of the needle channel so that the metal needle passes by or reaches the predefined position in the distal section;
   a handle assembly comprising a grip and an advancement member, the advancement member connected to the grip and configured to be movable relative to the grip, the advancement member configured to drive, under an action of an external force, the needle assembly to move in the axial direction of the needle channel; and
   a first monitoring device for monitoring whether the metal needle reaches the predefined position, the first monitoring device configured to generate a first target signal upon the metal needle reaching the predefined position.

2. The medical catheter of claim 1, wherein a cross-section of the distal section has a non-circular shape, and a circle inscribed on the cross-section of the distal section matches an outer diameter of the metal needle so that the metal needle is in a coaxial arrangement with the distal section when the metal needle is at least partially located in the distal section.

3. The medical catheter of claim 1 or 2, wherein the first monitoring device comprises a metal contact member that is disposed at the predefined position of the distal section so as to come into contact with an outer surface of the metal needle and be electrically connected to the metal needle to generate the first target signal.

4. The medical catheter of claim 3, wherein a ring-shaped first accommodating groove is defined in a wall of the distal section at the predefined position, wherein the metal contact member is a ring-shaped structure defining a first inner bore, wherein the metal contact member is mounted in the first accommodating groove, and wherein the first inner bore has a cross-section matching the cross-section of the distal section in terms of both shape and size.

**5.** The medical catheter of claim 3, wherein the catheter assembly comprises a catheter body, a tip electrode and a limiting member, the tip electrode disposed at a distal end of the catheter body, the tip electrode defining a second inner bore extending axially therethrough, the limiting member configured as an insulator disposed in the second inner bore, the limiting member defining a third inner bore extending axially therethrough, the third inner bore forming the distal section of the needle channel.

**6.** The medical catheter of claim 5, wherein a plurality of ridges extending in an axial direction of the limiting member is provided on a wall of the third inner bore, and the plurality of ridges are spaced apart across a circumference of the limiting member, wherein in a cross-section of the third inner bore, each of the ridges has a top located on a circle inscribed on the third inner bore.

**7.** The medical catheter of claim 5, wherein the needle assembly further comprises a first fluid tube connected at a proximal end thereof to the advancement member and at a distal end thereof to the metal needle, wherein
the metal needle, the first monitoring device and the tip electrode are electrically connected to an external device by separate wires, and the wires connecting to the first monitoring device and the tip electrode are provided in the catheter assembly.

**8.** The medical catheter of claim 7, wherein the first fluid tube defines a first lumen, wherein
the catheter body comprises an outer sleeve, a second fluid tube and an external adapter tube, the second fluid tube partially disposed in the outer sleeve, the second fluid tube extending in an axial direction of the outer sleeve, the second fluid tube connected at a proximal end thereof to the handle assembly, the second fluid tube configured to be stationary relative to the grip, the second fluid tube defining a second lumen for insertion of the first fluid tube therein so that a fluid channel is defined between the first fluid tube and the second fluid tube, the fluid channel separated from the first lumen, a wall of the second fluid tube at a proximal end thereof provided with a perfusion aperture in communication with the fluid channel, the external adapter tube in communication with the perfusion aperture, a distal end of the second fluid tube connected to a proximal end of the tip electrode, the second fluid tube in communication with the third inner bore.

**9.** The medical catheter of claim 1 or 2, wherein the first monitoring device comprises an infrared sensor disposed at the predefined position of the distal section.

**10.** The medical catheter of claim 1 or 2, wherein the handle assembly further comprises a manipulation member rotatably disposed at a proximal end of the grip, wherein the advancement member comprises a threaded rod having a distal end disposed inside the grip and connected to a proximal end of the needle assembly, the threaded rod configured to be circumferentially stationary relative to the grip and axially movable relative to the grip, a proximal end of the threaded rod spirally fitted to the manipulation member so as to be spirally rotatable.

**11.** The medical catheter of claim 10, wherein the threaded rod or the grip are provided thereon with graduation lines, at least some of which are visible from outside so as for allowing monitoring a movement distance of the threaded rod relative to the grip, or wherein the manipulation member is provided thereon with graduation lines so that an angle of rotation of the manipulation member relative to the grip is monitored, and a movement distance of the threaded rod relative to the grip is monitored based on a fixed conversion relationship between the angle of rotation and a displacement distance of the threaded rod.

**12.** The medical catheter of claim 10, further comprising a second monitoring device configured to produce a second target signal when the threaded rod moves relative to the grip, and wherein the movement distance of the threaded rod relative to the grip is calculated based on the second target signal.

**13.** The medical catheter of claim 12, wherein the second monitoring device comprises a first portion and a second portion, the first portion connected to the manipulation member and maintained axially stationary relative to the grip, the second portion connected to the threaded rod so as to be axially movable relative to the grip, the first and second portions cooperating with each other together to form a sliding rheostat connected in a circuit, the sliding rheostat configured to produce an electrical signal serving as the second target signal.

**14.** The medical catheter of claim 13, wherein the first portion comprises a metal base and a resistance wire, the metal base has an elongate structure extending in an axial direction of the threaded rod, the metal base connected at a proximal end thereof to the manipulation member so that the first portion is axially stationary relative to the grip, the resistance wire wound on an outer surface of the metal base, the second portion comprising a sliding tab in contact with the resistance wire, the sliding tab configured to slide on the resistance wire in response to movement of the threaded rod relative to the grip, to cause a change in a resistance of the sliding rheostat and hence in the electrical signal.

**15.** The medical catheter of claim 14, wherein a second accommodating groove is formed at a proximal end face of the threaded rod to receive the second portion, wherein the threaded rod further defines a channel extending axially, and the channel communicates with the second accommodating groove for insertion of the first portion.

**16.** A medical device, comprising a control unit and the medical catheter of any of claims 1 to 11, the control unit comprising a first signal acquisition module and an indication module, the first signal acquisition module communicatively connected to the first monitoring device and the metal needle and configured to collect the first target signal, the indication module configured to indicate, based on the first target signal, that the metal needle reaches the predefined position.

**17.** A medical device, comprising a control unit and the medical catheter of any of claims 12 to 15, wherein the control unit comprises a first signal acquisition module, an indication module, an electrical signal generation module, a second signal acquisition module and a calculation module, wherein
the first signal acquisition module is communicatively connected to the first monitoring device and the metal needle and configured to collect the first target signal, and the indication module is configured to indicate, based on the first target signal, that the metal needle reaches the predefined position,
the electrical signal generation module is configured to power the second monitoring device, the second signal acquisition module is communicatively connected to the second monitoring device and configured to collect the second target signal, the calculation module is configured to calculate, based on the second target signal, a movement distance of the advancement member relative to the grip.

**18.** The medical device of claim 17, wherein the control unit is configured so that the first signal acquisition module collects the first target signal and sends the first target signal to the indication module and the electrical signal generation module and hence the electrical signal generation module responsively powers the second monitoring device, and that the second signal acquisition module collects the second target signal and sends the second target signal to the calculation module and hence the calculation module responsively calculates the movement distance of the advancement member relative to the grip based on the second target signal.

**19.** The medical device of claim 18, wherein the control unit further comprises a storage unit in which a fixed distance from a proximal end of the first monitoring device to a distal end of the needle channel is pre-stored, wherein the calculation module is configured to calculate a difference between the movement distance and the fixed distance, and the difference is used to characterize an extended length of the needle coming out from the medical catheter.

**20.** The medical catheter according to any of claims 1 to 15, wherein during movement of the needle assembly driven by the advancement member away from the grip, monitoring on the movement distance of the advancement member relative to the grip is started at a time when the metal needle reaches the predefined position.

Fig. 1

Fig. 2

Fig. 3

220

311

310

323

320

322

500

Fig. 4

132

121

122

120

210

200

220

140

132a

132b

132c

132d

Fig. 5

120

Fig. 6

122

121

120

Fig. 7

A—A

120  122  123

Fig. 8

210  141

N

M

140

Fig. 9

412

410

411

Fig. 10

410

Fig. 11

123

122  120

410

L₁

Fig. 12

Fig. 13

Fig. 14

Fig. 15

## INTERNATIONAL SEARCH REPORT

| International application No. |
| --- |
| **PCT/CN2021/121398** |

### A. CLASSIFICATION OF SUBJECT MATTER

A61B 18/14(2006.01)i

According to International Patent Classification (IPC) or to both national classification and IPC

### B. FIELDS SEARCHED

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNPAT, CNKI, WPI, EPODOC: 微创电生理, 导管, 消融, 注射, 出针, 伸出, 长度, 深度, 距离, 监测, 传感器, 定位, catheter, inject+, ablation, position+, monitor+, sens+, length, depth, distance

### C. DOCUMENTS CONSIDERED TO BE RELEVANT

| Category* | Citation of document, with indication, where appropriate, of the relevant passages | Relevant to claim No. |
| --- | --- | --- |
| PX | CN 112220556 A (SHANGHAI MICROPORT EP MEDTECH CO., LTD.) 15 January 2021 (2021-01-15) claims 1-19, description paragraphs [0048]-[0080], figures 1-15 | 1-20 |
| Y | CN 110870791 A (SHANGHAI MICROPORT EP MEDTECH CO., LTD.) 10 March 2020 (2020-03-10) description, paragraphs [0051]-[0094], and figures 1-11 | 1-11,16,20 |
| Y | US 2020297239 A1 (ST. JUDE MEDICAL, ATRIAL FIBRILLATION DIVISION, INC.) 24 September 2020 (2020-09-24) description paragraph [0048], figures 7A-7B | 1-11, 16, 20 |
| Y | CN 105030325 A (BIOSENSE WEBSTER (ISRAEL), LTD.) 11 November 2015 (2015-11-11) description, paragraphs [0062]-[0066], and figure 4 | 5-8 |
| A | CN 107260300 A (CHANGZHOU LUNGHEALTH MEDICAL EQUIPMENT CO., LTD.) 20 October 2017 (2017-10-20) entire document | 1-20 |
| A | US 6004295 A (AN-GO-GEN INC.) 21 December 1999 (1999-12-21) entire document | 1-20 |

☐ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 December 2021** | **31 December 2021** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/121398**

| Patent document cited in search report | | | Publication date (day/month/year) | Patent family member(s) | | | Publication date (day/month/year) |
|---|---|---|---|---|---|---|---|
| CN | 112220556 | A | 15 January 2021 | CN | 112220556 | B | 12 March 2021 |
| CN | 110870791 | A | 10 March 2020 | CN | 110870791 | B | 03 September 2021 |
| US | 2020297239 | A1 | 24 September 2020 | WO | 2017184342 | A1 | 26 October 2017 |
| CN | 105030325 | A | 11 November 2015 | AU | 2020200098 | A1 | 30 January 2020 |
| | | | | JP | 2019213970 | A | 19 December 2019 |
| | | | | JP | 6896819 | B2 | 30 June 2021 |
| | | | | US | 2020085493 | A1 | 19 March 2020 |
| | | | | AU | 2015201670 | A1 | 05 November 2015 |
| | | | | EP | 3023071 | A2 | 25 May 2016 |
| | | | | EP | 3023071 | B1 | 04 July 2018 |
| | | | | US | 2017348046 | A1 | 07 December 2017 |
| | | | | US | 10687888 | B2 | 23 June 2020 |
| | | | | EP | 2942024 | A2 | 11 November 2015 |
| | | | | EP | 2942024 | B1 | 03 March 2021 |
| | | | | CA | 2888382 | A1 | 18 October 2015 |
| | | | | US | 2015297290 | A1 | 22 October 2015 |
| | | | | US | 9848943 | B2 | 26 December 2017 |
| | | | | CN | 110464451 | A | 19 November 2019 |
| | | | | JP | 2015205181 | A | 19 November 2015 |
| | | | | JP | 6595794 | B2 | 23 October 2019 |
| | | | | IL | 237704 | A | 31 July 2018 |
| | | | | CN | 105030325 | B | 11 October 2019 |
| CN | 107260300 | A | 20 October 2017 | EP | 3656326 | A1 | 27 May 2020 |
| | | | | WO | 2019015003 | A1 | 24 January 2019 |
| | | | | US | 2020146749 | A1 | 14 May 2020 |
| | | | | IN | 202017005986 | A | 19 June 2020 |
| US | 6004295 | A | 21 December 1999 | CA | 2241615 | A1 | 26 December 1998 |

Form PCT/ISA/210 (patent family annex) (January 2015)